# EUROPEAN PATENT SPECIFICATION

(11) **EP 4 619 052 B1**
(45) Date of publication and mention of the grant of the patent: **16.09.2026**
(21) Application number: 23806309.3
(22) Date of filing: 16.11.2023
(51) Int. Cl.: A61L 27/16, A61L 27/50

(54) **ARTIFICIAL VASCULAR PROSTHESES MADE WITH POLYVINYL ALCOHOL AND METHOD FOR OBTAINING THE SAME**
KÜNSTLICHE GEFÄSSPROTHESEN AUS POLYVINYLALKOHOL UND VERFAHREN ZU IHRER HERSTELLUNG
PROTHÈSES VASCULAIRES ARTIFICIELLES FABRIQUÉES À PARTIR D'ALCOOL POLYVINYLIQUE ET LEUR PROCÉDÉ D'OBTENTION

(30) Priority: 17.11.2022 EP 22306694
(43) Date of publication of application: 24.09.2025
(73) Proprietor: Institut National de la Santé et de la Recherche Médicale, 75013 Paris (FR); UNIVERSITE PARIS 13 PARIS NORD, 93430 Villetaneuse (FR); Université Paris Cité, 75006 Paris (FR)
(72) Inventor: CHAUBET, Frédéric, 93430 VILLETANEUSE (FR); GERNEZ, Lucie, 93430 VILLETANEUSE (FR); BATAILLE, Isabelle, 93430 VILLETANEUSE (FR)
(74) Representative: Plasseraud IP
(86) International application number: PCT/EP2023/082141
(87) International publication number: WO 2024/105208

(56) References cited:
- US-A1- 2016 030 165
- US-A1- 2016 361 462
- US-B2- 11 028 502

## Description

### FIELD OF THE INVENTION

The present invention relates generally to the field of medicine and, in particular, to artificial vascular prostheses for treatment of vascular diseases. More specifically, the present invention relates to polyvinyl alcohol (PVA) tubular hydrogels cross-linked with molecules having a therapeutic interest (e.g., heparin and/or dextran), having enhanced hemo-compatibility and mechanical properties, and to a simple method for obtaining these. The PVA vascular prostheses described herein are particularly useful in applications such as the replacement of small-diameter native blood vessels that have been damaged or diseased.

### BACKGROUND OF THE INVENTION

Vascular prostheses, commonly referred to as vascular grafts, are tubular constructs typically used to replace, repair, or bypass damaged, occluded or diseased portions of blood vessels in the course of a surgical procedure.

The use of vascular grafts is important in the context of patients with cardiovascular diseases, which represent a leading cause of death in developed countries and a significant disease-related cost for their health systems.

Cardiovascular diseases, notably stroke, transient ischemic attack and atherosclerosis, frequently result in a narrowing of the vascular lumen of the affected arteries with dramatic effects. At present, there are therapeutic solutions to these phenomena such as endovascular techniques. However, they remain insufficient in terms of durability. In fact, it is often necessary to replace the damaged arterial segment or to perform a bypass with a suitable vascular substitute.

Grafts can be either natural or synthetic (or artificial). Synthetic grafts are routinely used for large vessel replacement (*i.e.,* those having a diameter greater than 6 mm) and are normally composed of synthetic or natural polymers, optionally mixed with molecules having a therapeutic activity. Several biocompatible polymers which can be used in synthetic grafts have been described (also referred to as 'hydrogels', because of their capacity to swell in water). However, for small-diameter vessel replacement (*i.e.,* those having a diameter equal or less than 6 mm), natural grafts such as autologous veins are still preferred because they have superior bio-compatibility and mechanical properties, more closely matching those of the native vessels.

A number of methods have been proposed over the years in order to improve the bio-compatibility of synthetic vascular grafts. The first of these methods is endothelialization, which consists in the coating of the vascular grafts with endothelial cells. Under normal physiological conditions, the endothelium guarantees anti-thrombogenic properties to the luminal surface. Another method consists in the use of natural polymers, which have excellent bio-compatibility and enhanced biological properties when compared to the synthetic polymers, but insufficient mechanical properties when used alone. Natural polymers traditionally used include chitosan, dextran, gelatin and collagen. These natural polymers are known to promote cell adhesion and proliferation (in addition to other properties). Used in the design of vascular prostheses, they promote endothelialisation. Other methods use biomolecules with anti-thrombotic properties such as heparin, dextran and nitrogen monoxide. These molecules are incorporated into the polymers used to make the vascular grafts, either by covalent or non-covalent linkage. They act as a reservoir for the sustained release of the anti-thrombogenetic agents, thereby greatly reducing the risk of thrombotic events, which is one of the main issues encountered in the use of artificial vascular grafts. Additionally, they overcome the significant drawbacks of heparin therapy.

A further method consists in the use of vascular grafts made of fibers, which can be obtained by electrospinning process. The structure of the electrospun fibers resembles the natural environment of the endothelial cells *(i.e.,* the extracellular matrix) and the size and orientation of the fibers seem to favourably influence the behavior of these cells. An example of vascular grafts made of fibers and of a method for their fabrication are described in US 11,028,502 B2.

The replacement of small-diameter vessels is, at present, performed only with autologous vessels, *i.e.* vessels taken directly from the patient (usually the saphenous vein or the internal mammary artery). However, autologous grafts are not always available because patients requiring this type of operation frequently have a smaller pool of healthy vessels that can be used for grafting due to their vascular condition and their taking may lead to donor site complications. Vessels taken from another human donor, also called allografts, or vessels taken from animal donors, or heterografts, are also used in some cases, but they carry the risk of immunogenicity and are prone to degeneration over time. The materials used in large-diameter synthetic prostheses often do not perform well when used in the replacement or bypass of small vessels. For example, polyethylene terephthalate (Dacron^{®}) and polytetrafluoroethylene (Teflon^{®}) are not suitable for small-diameter grafts and material-related problems such as stenosis, thromboembolization, calcium deposition, and infection have been described. As a consequence, clinicians are often faced with the unavailability of vascular material. In fact, no prosthesis with a diameter of less than 6 mm has met, to date, the mechanical and biological conditions for clinical use.

Hence, there is a clinical need for small-diameter vascular grafts with high mechanical properties and hemocompatibility. Such vascular grafts could be used for long-term as well as short- or medium-term replacements of diseased or damaged, or even destroyed, small-diameter vessels. In particular, there are emergency situations in which the availability of ready-to-use vascular grafts could be of crucial importance for the patient in need. Such a ready-to-use vascular graft would allow tissue vascularisation to be maintained while the patient is waiting for a permanent therapeutic graft. The specifications for this type of temporary implant would be less demanding than for a long-lasting prosthesis.

The present application pertains to a novel method for obtaining functionalized small-diameter vascular prostheses with suitable mechanical properties and satisfactory hemocompatibility. The vascular prostheses obtained by this method are based on the use of polyvinyl alcohol (PVA) to provide adequate mechanical properties to the prosthesis, possibly coupled to the use of natural polymers such as dextran and heparin to cover the luminal surface of the prosthesis in order to improve its hemocompatibility. Polyvinyl alcohol is a synthetic polymer which provides a material with mechanical properties that can be adjusted to those of native arteries. These properties depend, in particular, on the cross-linking protocol. It is an inexpensive and widely used polymer, particularly by the food and biomedical industries. Dextran and heparin have long been used in the vascular field, and are still used today, in particular to improve the hemocompatibility of implants by reducing their thrombotic effect. The method described herein has the additional advantage of being simple and compatible with industrial production.

### SUMMARY OF THE INVENTION

As shown in the experimental part of the present application, the inventors have conceived a simple and reliable method of obtaining an artificial vascular prosthesis.

Accordingly, a first aspect of the present invention pertains to a method of obtaining an artificial vascular prosthesis comprising the physical reticulation of polyvinyl alcohol (PVA) by a single cycle of freezing/thawing and drying.

More particularly, the method comprises the steps as defined in claim 1.

The method is particularly conceived for obtaining small-diameter vascular prostheses, *i.e.* those having an internal diameter equal to or less than 6 mm.

The present invention also pertains to the functionalization of the PVA tubular hydrogel with one or more therapeutic molecules, in particular with the aim to improve the hemocompatibility of the resulting artificial vascular prosthesis.

According to some embodiments, the functionalization is performed by cross-linking carboxymethyldextrane and/or heparin *via* the formation of an amide bond between their carboxylic functionalities and primary amine groups created on the intraluminal surface of the PVA tubular hydrogel.

The amination protocol developed by the inventors allows to attain a high degree of substitution of the hydroxyl functions. In addition, the amination protocol results in the uniform distribution of the amine groups along the intraluminal surface of the PVA tube and limits the amination to this surface, avoiding deleterious alterations of the mechanical properties of the prosthesis.

Accordingly, one aspect of the invention relates to the amination with an amination reagent such as 4-aminobutyraldehyde diethyl acetal (4-ABA), with the aid of a system for its continuous circulation, which guarantees the uniform and superficial amination of the intraluminal surface.

Another aspect of the invention relates to the PVA tube or the functionalized vascular prosthesis obtained by the method of the invention.

According to some embodiments, the PVA tube and the functionalized vascular prosthesis are characterized by values of compliance and induced hemolysis that respect the actual requirements for *in vivo* implantation.

### BRIEF DESCRIPTION OF THE DRAWINGS

**Figure 1****:** General chemical formula of PVA. The proportion of n and m determines the level of deacetylation. A PVA with a 99+% level of hydrolysis essentially contains only the n monomer.
**Figure 2****:** (A) Representation of a mold according to the present invention, characterized by an external diameter d1, an internal diameter d2, a thickness t = d2 - d1 and a length L, (B) transversal cross-section of the mold, showing the meaning of d1 and d2.
**Figure 3****:** X-ray powder diffraction profile of a tubular PVA hydrogel formulated with 9.7% (w/w) of PVA. Both curves, experimental data and computed data, are overlaid on each other.
**Figure 4****:** Nitrogen proportion (% w) in PVA tubular gels as such (PVA), or treated by one (PVA-1), two (PVA-2), three (PVA-3) or four (PVA-4) cycles of amination (of 30min). (Statistical analysis: ANOVA PVA vs aminated PVA, ****: p<0,0001).
**Figure 5****:** Fluorescence microscopy images of CMD-FITC grafted PVA tube cross section (x10). A. Transmitted light image. B. Fluorescence image. C. Merge image. Dotted circles define the walls of the tube.
**Figure 6****:** Suturability of tubes with different PVA concentrations and surface modifications.
**Figure 7****:** Compliance of tubes having 9.7%, 8%, 7% and 5% (w/w) PVA. The compliance is calculated between 60 and 140 mmHg. % = w/w. The NaOH concentration used for the formulation of the tubes is 0.2 M. The results are the mean ± standard deviation standard deviation (n=3). Univariate ANOVA statistical test. ***: p < 0.001; **: p < 0.01. ND: not determined.
**Figure 8****:** Hemolysis induced by different formulations of PVA tubes. All tubes are formulated with a 9.7% PVA solution. The negative control is PBS 1X, and the positive control (not shown) is a titron solution. Statistical analysis one-way ANOVA vs negative control. **: p<0,01; *: p<0,05.

### DETAILED DESCRIPTION OF THE PREFERRED EMBODIMENTS

According to a first aspect, the present invention pertains to a method of obtaining an artificial vascular prosthesis.

"Artificial vascular prosthesis" herein designates a tubular construct used to replace, repair or bypass damaged, occluded or diseased portions of blood vessels. More in particular, an artificial vascular prosthesis according to the invention is a polyvinyl alcohol (PVA) tubular hydrogel obtained by a method of physical reticulation of partially deacetylated PVA with a concentrated alcaline solution, optionally having the internal surface functionalized with therapeutic molecules capable of improving the hemocompatibilty of the prosthesis, especially to enhance its anti-thrombogenic properties.

According to the invention, the artificial vascular prosthesis obtained by the claimed method is appropriate for its function as vessel replacement in the human or animal body, *i.e.* it must have appropriate biological (particularly in terms of hemocompatibility) as well as mechanical properties (particularly in terms of compliance and suturability), closely matching those typical of the native vessels to be replaced.

The method of the present invention is particularly appropriate for obtaining small-diameter artificial vascular prostheses, *i.e.* vascular prostheses having an internal diameter equal to or smaller than 6 mm.

For purposes of describing the present invention, the terms "vascular graft", "vascular prosthesis" and "vascular substitutes" are interchangeably used in describing the invention.

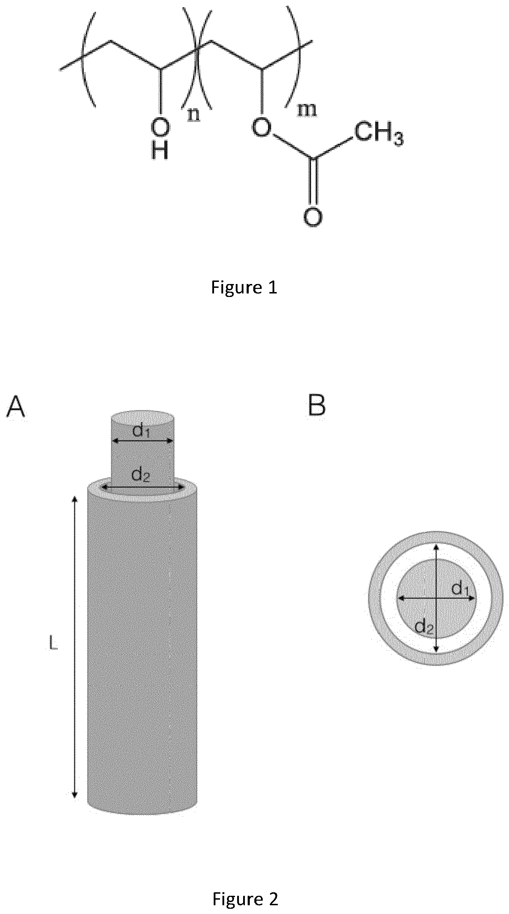

As used herein, the terms "polyvinyl alcohol" or "PVA" refer to a water soluble polymer synthesized by hydrolysis of the polyvinyl acetate in alkaline environment. The term PVA refers to a variety of linear polymers characterized by a variable degree of polymerization and of hydrolysis (*i.e.,* deacetylation). These two factors have a direct impact on the solubility of PVA and other physicochemical properties. The degree of polymerization determines the molecular weight and viscosity of the resulting solution. The degree of hydrolysis indicates the extent of the conversion of the acetate moieties of the polyvinyl acetate to hydroxyl moieties. In the case of complete hydrolysis, the resulting PVA contains no acetate group (*i.e., m* = 0 in the PVA chemical formula of Figure 1). In case of partial hydrolysis of the polyvinyl acetate ester, the resulting PVA contains a variable amount of acetate groups (*i.e.,* there is a certain proportion of *n and m* in the PVA chemical structure represented in Figure 1). The degree of hydrolysis of commercially available polyvinyl alcohols is in the range of about 60% to about 99.9%, while the molecular weight may range from about 10 kDa to about 250 kDa.

The polyvinyl alcohol used in the method according to the invention has a deacetylation level between 80% and 90%, preferably between 85% and 88%, and a molecular mass in the range between 85 kDa and 130 kDa.

An important advantage of the method according to the invention is that the partially deacetylated PVA has a higher water solubility compared to a fully hydrolysed PVA, so that it is easier to obtain a homogenous pre-gel solution.

Another characteristic of the PVA which is important in the context of the present invention is that it can be cross-linked, or reticulated (the two terms are used interchangeably in the present text) either chemically (e.g., by using glutaraldehyde, formaldehyde, sodium trimetaphosphate or STMP) or physically (by freeze/thaw cycles) to form hydrogels. The control of the reticulation of the PVA allows the mechanical properties of the resulting PVA hydrogel to be adjusted according to the desired application.

In the context of the present invention, an original physical process of reticulation of PVA is used, which combines a unique cycle of freeze/thaw of the pre-gel PVA solution, followed by a step of drying (or dehydration).

The advantage of using a physical over a chemical reticulation protocol is that in chemical reticulation, the cross-linking reagent can remain inside the reticulated hydrogel at the end of the process, which can be (cyto)toxic once the prosthesis is implanted. In addition, cross-linking with a chemical such as STMP introduces phosphate groups between the PVA chains, which could promote calcification of the prosthesis once implanted.

According to the invention, the concentration of PVA in the pre-gel solution is in the range from 4% to 10% (w/w). For example, final concentrations of PVA in the pre-gel solution are: 9.7%, 8%, 7% and 5% (w/w). This is obtained, for example, by using a 10% (w/w) partially deacetylated PVA solution.

By "alcaline aqueous solution" is herein meant an aqueous solution of a water soluble alkali. The alkali is any metal hydroxide with a high degree of dissociation in water and which confers a pH equal to or greater than 12 to the resulting solution. The ability of a metal hydroxide to completely (or almost completely) dissociate in water and, therefore, to behave as a strong base, depends on the nature of the metal. Certain metal hydroxides completely dissociate when dissolved in water and behave, therefore, as strong bases. Other metal hydroxides, on the contrary, only weakly dissociate when dissolved in water and only have a weak base character.

According to the present invention, the alcaline aqueous solution preferably is a solution of a strong base. For example, the alkaline aqueous solution is a solution of NaOH, e.g. an aqueous solution of NaOH 6.25 M. Another alcaline solution which can be used according to the invention is KOH.

According to the invention, the partially deacetylated PVA and the alcaline solutions are mixed to form a pre-gel solution. Mixing can be performed by dropwise addition of the alkaline solution to the partially deacetylated PVA solution placed under stirring.

According to the invention, the final concentration of hydroxyl ions (OH⁻) in the pre-gel solution is in the range from 0.15 M to 0.30 M, preferably less than 0.20 M (e.g. 0.17 M), so as to obtain a transparent solution.

After the partially deacetylated PVA and the alcaline solutions are mixed, the pre-gel solution can, for example, be placed under agitation and sonicated until a transparent and homogenous bubble-free viscous solution is obtained.

A transparent prosthesis can thus be obtained, which represents an important aspect of the present invention in that it allows the internal inspection for possible defects. The characterization by X-ray Powder Diffraction explains the transparency of the prosthesis by the presence of small crystallites of nanometer size.

According to the invention, the pre-gel solution is introduced into a mold comprising two coaxial cylinders, a smaller cylinder with an external diameter d1 and a larger cylinder with an internal diameter d2, and a thickness t = d2 - d1 of the tube before the drying and the hydration steps. Figure 2 shows the geometry of the mold and the meaning of d1, d2, t and L.

According to the invention, d1 is in the range from 0.8 mm to 6 mm, d2 is in the range from 3.0 mm to 9.0 mm and the thickness is in the range from 0.9 mm to 3.0 mm, preferably in the range from 1.5 mm to 2.5 mm. Therefore, an advantage of the method of the present invention is that it allows to obtain tubes having different diameter and thickness values, depending on the specific need. Of course, the method according to the invention can also be performed with molds having a geometry not limited to the tubular part. For example, the mold can create a tube with a tab for increasing the ease of manipulation during the subsequent steps of the method. The skilled person can adapt the geometry of the mold without any difficulty.

According to the invention, the mold containing the pre-gel solution is frozen to obtain a tube of frozen PVA hydrogel. The freezing can be performed, for example, by placing the mold containing the pre-gel solution for 1 hour at a temperature between -15°C and -40°C, e.g., for 1 hour at -20°C. Depending on the size of the mold and other experimental variables, the time required to completely freeze the pre-gel solution inside the mold may need to be adjusted. The freezing can be performed by keeping the mold e.g, vertically in order to avoid the formation of defects and/or bubbles in the final PVA tubular hydrogel.

As used herein, the term "hydrogel" refers to a cross-linked polymeric material which has a three-dimensional network, that is insoluble in water and which is obtained by physical reticulation of partially deacetylated polyvinyl alcohol (PVA) by a process comprising a cycle of freezing/thawing and drying. Such hydrogel can hold a large amount of water. Usually, the majority of the mass of the hydrogel (typically greater than about 80%) is that of the entrapped water. The water to PVA ratio of the hydrogel is important in that it determines, among other factors, its mechanical properties and chemical stability. Another aspect of the PVA hydrogel important in the context of the present invention is the presence of pendant hydroxyl groups on the surface of the polymer which are free and which provide attachment sites for a variety of therapeutic molecules.

During the freezing step, the pre-gel solution undergoes a physical transformation that is important for the final properties of the vascular prosthesis. The freezing process can be considered completed when the center of the tubular gel is completely frozen. The freezing step allows the pre-gel aqueous solution to solidify, which results in the formation of regions of high polymer concentration, thus inducing the generation of crystallites, *i.e.* crystalline ordered domains (of nanometer size). This is the first step of the physical reticulation and, when the freezing is completed, the original polymer is already mostly reticulated. However, the reticulation will continue in the following steps of the present method (particularly, during the drying), further changing the properties of the hydrogel.

According to the invention, once the freezing is completed the part of the mold comprising the larger cylinder is removed (outer part of the mold). The removal of the part of the mold comprising the larger cylinder can be performed, for example, when the interior of the tube is still frozen and the external surface of the tube starts to melt and can easily be detached from the outer part of the mold.

According to the invention, the drying (or dehydration) of the PVA tubular hydrogel is done for at least 24 hours, preferably 24-72 hours at a temperature in the range from 4°C to 20°C, preferably in the range from 4°C to 10°C and for 18-48 hours at a temperature in the range from 30 to 60°C, preferably around 50°C.

At the end of the drying step, the hydrogel has further changed its properties. This is because, during the dehydration step, the reticulation of the polymer network has continued. In particular, the water is progressively eliminated during this step, which allows the polymer chains to come together and to form new crystallites. The increased level of reticulation has the effect to further rigidify the tubes. Therefore, the drying step is necessary not only because after the freezing cycle the reticulation is weak, the gel is sticky and cannot be handled, but also because after the freezing step the gel still does not possess the required mechanical properties.

The drying step can for example be performed in a ventilated or non-ventilated refrigerator.

According to the invention, at the end of the drying step, the PVA tubular hydrogel is placed in an appropriate buffer at room temperature for at least 10 minutes. The hydration of the tube is preferably performed in a buffer in the pH range 6.5-7.5, for example, in PBS 1X or in any physiological buffer. The time of hydration can vary between 10 and 120 minutes, for example 90 minutes, depending on the time necessary for the tube to reach its swelling equilibrium with the surrounding solution. The temperature at which the hydration is conducted can vary between, for example, 15°C and 30°C. According to the invention, the part of the mold comprising the smaller cylinder (inner part of the mold) is removed after hydration. To remove any possible trace of the alkaline solution in the tube, the PVA tubular hydrogel can be repeatedly washed at the end of the hydration step, e.g., by rinsing it 3 times with PBS 1X.

The PVA tubular hydrogel is then characterized by a swelling ratio between 149% and 525%.

The PVA tubular hydrogel can be stored in PBS 1X at 4°C and protected from light.

According to a particular embodiment of the invention, the tube obtained by the above method has a compliance in the range from 7 to 18%, preferably from 11 to 15% as assessed by measuring the internal diameter while pressures ranging from 60 mmHg to 140 mmHg are applied. The internal diameter can be measured with the aid of any imaging system able to provide a measure that is sufficiently precise and accurate to differentiate between the diastolic and the systolic vessel diameters, such as ultrasonography (US) imaging, the oscillometric method (NIBP), the continuous non-invasive arterial pressure method (CNAP) and any other appropriate method known to the person of skills in the art.

Indeed, as described in the experimental part which follows, the resulting vascular prosthesis demonstrated excellent mechanical properties, inter alia in terms of compliance.

According to another particular embodiment of the invention, the method further comprises a step of contacting the intraluminal surface of the tube with a solution of an amination reagent to covalently link primary amino groups to said intraluminal surface. According to a particular embodiment, the amount of primary amino groups expressed as grams of nitrogen per 100 grams of dried tube is at least 0.2%, for example 2% or more after the amination step.

By "amination" is herein meant an organic chemistry reaction by which a primary amino group is introduced at the surface of PVA. In the context of the present invention, the insertion of the primary amino groups is performed by an amination reagent.

As used herein, the term "amination reagent" refers to any chemical reagent capable of performing the amination reaction. Preferably, the amination reagent used to perform the invention leads to a degree of substitution of at least 0.2 to 2%, as described above. In the context of the present invention, the amination reagent can be an amino acetal, for example a chemical reagent in the class of aminoalkylaldehyde alkyl acetals. The skilled person can choose the amination reagent and the conditions of the amination step (concentration of the reagent, duration of the cycles of amination and number of said cycles) to obtain an appropriate degree of substitution.

Accordingly, in a particular embodiment, the amination reagent is 4-aminobutyraldehyde diethyl acetal (4-ABA).

In a particular embodiment, the amination of the intraluminal surface of the tube is performed by contacting the intraluminal surface of the tube with a solution of 4-ABA, preferably at a concentration in the range from 0.5 M to 2.0 M, preferably in the range from 0.8 M to 1.0 M, at pH < 1.

According to another aspect of the invention, the contact between the amination reagent and the intraluminal surface of the tube is performed by circulating a solution of the amination reagent and is followed by the rinsing of the intraluminal surface of the tube. The circulation of the solution of the amination reagent can be performed, for example, with the aid of a peristaltic pump.

The rinsing of the intraluminal surface of the tube can be performed, for example, with an aqueous solution of PBS 1X.

In a particular embodiment, the solution of the amination reagent is circulated through the tube for several cycles, e.g., 2, 3, 4 or more, each followed by the rinsing of the intraluminal surface of the tube. Still according to this embodiment, the solution of the amination reagent is preferably circulated for 1 or 2 cycles in order to obtain the desired technical effect, which is the increase of the number of primary amino groups linked on the PVA intraluminal surface while avoiding, at the same time, the amination reagent to penetrate into the material more than some tens of micrometers. By performing amination cycles of limited duration, followed by rinsing steps, the diffusion of the reaction solution is limited within no more than some tens of µm of the tube wall. In fact, any in-depth alteration of the PVA structure could lead to deleterious alterations of the tube, especially concerning its mechanical properties. According to a particular embodiment, the reaction cycles are of short duration (e.g., 30 min) and are repeated several times, interspersed with rinses, which allows the amination to be concentrated on the luminal surface of the PVA tube only. With this protocol, the swelling of the tubes is greatly reduced, even with 4 successive aminations (4 x 30 min interspersed with 1 hour of rinsing). In this context, the use of a peristaltic pump has several advantages in that it allows: (i) the contact of the solution of the amination reagent with the luminal surface of the PVA tube only, (ii) gentle flow conditions (*i.e.* low flow rate), (iii) continuous circulation (*i.e.* always fresh amination reagent available for the reaction), (iv) homogeneous distribution of the solution of the amination reagent and (v) a simplicity of use. Therefore, the peristaltic pump represents an optimal system to carry out the amination of the PVA intraluminal surface.

According to another embodiment, the method further comprises a step of functionalizing the intraluminal surface of the tube by covalently or non-covalently linking molecules having a therapeutic interest to said surface. Non-covalently linking is preferably related to strong and numerous ion-pairs formation between positive amine functions and negative groups of the molecule used for functionalization. In particular, carboxymethyldextran and heparin, which are anionic polymers, can efficiently be bound to the luminal surface by such non-covalent interactions.

As used herein, the term "cross-link" refers to a chemical bond linking one molecule to another (either covalently or non-covalently), wherein the linked molecules are usually two or more similar or dissimilar polymers. Such polymers can be either non-natural (synthetic) or natural polymers.

As used herein, the term "cross-linking" refers to a chemical reaction to form cross-links as above-described. Allowing the formation of cross-links between the chains of polymers, the cross-linking reaction promotes a change in the polymers' physical properties and the reaction can be used to fine-tune the properties of the final, cross-linked product.

In the present application, for purposes of describing the invention, the terms "cross-linking", "functionalization" and "grafting" are interchangeably used when describing the linking of therapeutic molecules to the PVA tube.

Molecules having a therapeutic interest can be selected from the group consisting of: anti-thrombogenic agents, anti-bacterial agents, anti-viral agents, growth factors, cytokines and mixtures thereof. The skilled person can adapt the molecules grafted to the PVA tube, and the grafting method (e.g., covalent or non-covalent grafting) depending on the medical needs (Pagel, M.; Beck-Sickinger, AG. Multifunctional biomaterial coatings: synthetic challenges and biological activity. Biological Chemistry, 2017, 398(1), pp3-22).

In the context of the present invention, the molecules having a therapeutic interest comprise carboxymethyldextran and/or heparin.

As used herein, the term "heparin" refers to highly sulfated and polydisperse glycosaminoglycans with molecular weights ranging from 5 kDa to 40 kDa, having a complex structure consisting of repeating disaccharide units consisting of uronic acid residues (L-iduronic (IdoA) or D-glucuronic acid (GlcA)) and N-acetyl-D-glucosamine. The naturally occurring heparin, also known as unfractionated heparin (UFH), is to be distinguished from the Low Molecular Weight Heparin (LMWH) which, in contrast, consists of only short chains. LMWHs can vary in size from 2 kDa to 12 kDa, with an average molecular weight of less than 8 kDa. LMWHs are obtained by various methods of fractionation or depolymerisation of polymeric heparin in which, for example, heparin is chemically or enzymatically depolymerised. Both types of heparin, UFH and LMWH, belong to the class of anticoagulants. Heparin-like molecules such as LMWHs are often used because of the longer half-life, the controlled chemical composition and reduced fewer side-effects.

In the context of the present invention, low molecular weight heparin is preferably used. The molecular mass of the low molecular weight heparin is preferably in the range between 3.5 kDa and 6.5 kDa.

As used herein, the term "carboxymethyldextran" or "CMD" refers to a polysaccharide that can be produced by reacting dextran fractions with an activated carboxymethyl derivative in alcaline media. This leads to the introduction of O-carboxymethyl groups along the dextran chain. The content of carboxylic functions can vary and different carboxymethyldextran having variable degrees of substitution may be obtained. Therefore, there exists a number of carboxymethyldextran products characterized by different molecular weight and vaying degree of substitution.

In a particular embodiment, carboxymethyldextran and/or heparin are covalently and/or non-covalently linked to the aminated intraluminal surface of the PVA tube. According to a particular embodiment, carboxymethyldextran and/or heparin are covalently linked to the aminated intraluminal surface of the PVA tube in a ratio comprised between 1:2 and 2:1, for example around 1:1 (expressed as moles - COOH/moles -COOH).

In a particular embodiment, the functionalization of the aminated intraluminal surface of the tube is performed by circulating a solution of the molecule of therapeutic interest, followed by the rinsing of the intraluminal surface of the tube.

The circulation of the solution of the amination reagent can be performed, for example, with the aid of a peristaltic pump as already described. The solution of the amination reagent can be circulated with the peristaltic pump for 1 cycle of functionalization during several hours (e.g., 6 to 12 hours). The rinsing of the intraluminal surface of the tube can be performed, for example, with an aqueous solution of PBS 1X.

For example, the cross-linking of the aminated intraluminal surface of the tube is performed by circulating, with the aid of a peristaltic pump, an aqueous solution of carboxymethyldextran 6.0 x 10⁻² M and heparin 3.4 x 10⁻⁴ M (1:1 ratio).

Similarly to the amination, the functionalization protocol allows for a uniform cross-linking of the therapeutic molecules, which is limited to the surface of the PVA tubes.

In a particular embodiment, the functionalization of the intraluminal surface of the tube by covalently linking one or more therapeutic molecules is performed by a coupling system. For example, the classical coupling system is 1-Ethyl-3-(3-Dimethylaminopropyl)Carbodiimide (EDC)/N-HydroxySuccinimide (NHS).

EDC and other carbodiimides are zero-length crosslinkers allowing the direct conjugation of carboxylates to primary amines without becoming part of the final crosslink (an amide bond) between the target molecules. N-hydroxysuccinimide (NHS) or its water-soluble analog (Sulfo-NHS) is often included in EDC coupling protocols to improve efficiency or to create a more stable, amine-reactive intermediate. The mechanism is based on the activation by the EDC/NHS system of the carboxylic functions of the polysaccharides (carboxymethyldextran and/or heparin), so that the activated carboxylic groups can react with the primary amines grafted to the luminal surface of the tubes forming amide bonds.

EDC cross-linking reactions are preferably performed in conditions devoid of extraneous carboxyls and amines. Acidic (pH 4.5 to 5.5) MES buffer (4-morpholino-ethane-sulfonic acid) is most effective, but phosphate buffers at pH ≤ 7.2 are also compatible with the reaction chemistry.

For example, the cross-linking of the aminated intraluminal surface of the tube is performed by circulating, with the aid of a peristaltic pump, an aqueous solution containing carboxymethyldextran 6.0 x 10⁻² M and heparin 3.4 x 10⁻⁴ M (1:1 ratio), to which EDC and NHS are added to a final concentration of 0.13 M and 0.22 M, respectively, and having a pH between 6.5 and 7.5.

The inventors demonstrated the effective grafting of carboxymethyldextran by confocal microscopy measurements with fluorescent CMD (CMD-FITC) (not shown). Flow graft resistance measurements also demonstrated that the cross-linked therapeutic molecules, whether linked covalently or non-covalently, withstand the typical coronary flow for at least 35 hours, indicating that the coating is mechanically resistant to the blood flow.

The invention also pertains to the PVA tube or the functionalized vascular prosthesis.

According to a particular embodiment, the PVA tube or functionalized vascular prosthesis has a compliance in the range between 11 and 15%/100 mmHg as assessed by measuring the internal diameter, for example with ultrasonography imaging or any other appropriate method as above-described, while pressures ranging from 60 mmHg to 140 mmHg are applied.

In terms of mechanical properties of the functionalized vascular prosthesis, the assessment of the compliance demonstrates that the prostheses have a compliance comparable to that of healthy arteries, while the suturability measurements show that they well resist to the suture in terms of supported mass.

According to a particular embodiment, the PVA tube or the functionalized vascular prosthesis is characterized by its hemocompatibility, quantified by an induced hemolysis below 5%, preferably below 2%.

As used herein, the term "hemocompatibility" is to be understood as a measure of the thrombotic response induced by the artificial vascular prosthesis when it comes into contact with blood. The interaction between a material or device and the blood leads to cellular as well as humoral reactions, which can result in an unwanted inflammation and activation of coagulation and/or fibrinolysis at the implantation site. The hemocompatibility reflects the extense of this reaction. Of particular importance in the context of the present invention is the ability of the artificial vascular prosthesis to induce thrombus formation, which is one of the most important unwanted side effects.

When producing an artificial vascular prosthesis, the aim is also to avoid that it adversely interacts with any blood components. Thus, a successful vascular prosthesis must be produced of a material which causes no or extremely reduced levels of hemolysis.

As used herein, the term "hemolysis" refers to the rupturing (lysis) of erythrocytes and the release of their contents in the surroundings. In particular, in the context of the present invention, the term hemolysis refers to the capacity of an artificial vascular prosthesis to induce the rupture of the erythrocytes.

The hemocompatibility of the artificial vascular prosthesis can therefore be measured by its induced hemolysis.

Platelet activation is another way to measure the hemocompatibility of an artificial vascular prosthesis. The platelets activation test is based on the fact that, in normal conditions, the adhesion and activation of platelets is prevented by an healthy endothelial monolayer with antithrombogenic properties. A damaged endothelium triggers a series of events resulting in the adhesion of circulating platelets to form a seal at the damaged area. In a similar way, the implantation of an artificial vascular prosthesis can result in an undesired activation of platelets and consequently lead to thrombotic complications. Thus, platelet activation is an important hemocompatibility test.

In terms of biological properties of the functionalized vascular prosthesis, the measured hemolysis induced by the tubes shows that the prostheses are only slightly hemolytic and always within the required limits for implantation, and platelets activation tests show that the functionalized prostheses do not induce major platelet activation within the incubation time during which they are tested.

Therefore, the functionalization of the intraluminal surface of the PVA tubular hydrogel further improves the hemo-compatibility in terms of its anti-hemolytic and anti-thrombogenic properties with no detriment to the mechanical properties of the prosthesis.

### EXAMPLES

### Example 1: Preparation of reticulated PVA tubular hydrogels

In this Example, the experimental protocol to obtain the PVA tubular hydrogels is described, in which partially hydrolysed PVA is physically reticulated by using concentrated sodium hydroxide (NaOH). The characterization of the PVA tubular hydrogels by X-ray Powder Diffraction (XRPD) and Scanning Electron Microscopy (SEM) is also reported.

### Materials and Methods

### Materials

Partially hydrolysed polyvinyl alcohol (MW: 88 130 kDa; deacetylation level: 86.7-88.7 mol% hydrolysis) was purchased from Sigma-Aldrich. Sodium hydroxide was obtained from Carlo Erba. The water used, known as "ultrapure", was purified to 18.2 MΩ·cm with the Purelab Flex system.

### Protocol for the preparation of reticulated PVA tubular hydrogels

A solution of 10% (w/w) partially hydrolysed PVA in water was prepared by dissolving 10 g of PVA in 90 g of ultrapure water (UP). The solution was placed in a water bath at 70°C under stirring until it was completely dissolved. A pre-gel solution was made by adding 6.25 M sodium hydroxide drop by drop under stirring to the 10% (w/w) PVA solution to obtain a 5 to 10% (w/w) PVA and 0.17M NaOH pre-gel solution. The pre-gel solution was left to stir at 350 rpm for 10 min at room temperature and then poured into molds constituted of a syringe body (external mold) and a needle (rode). Syringes of 1mL (4mm diameter), 0,5mL (3,5mm diameter) and 0,3mL (3mm diameter) and needles of 21G (0,8mm diameter) to 14G (2,1mm diameter) were used. The needle was centered with seals. The use of syringes and needles with different diameters allowed to obtain tubes with various diameter and thickness. The assembly was kept vertically at -20°C for 1 h. The mold was then immersed for about 20 seconds in a water bath at 35°C to separate the rod with the gel from the body of the mold. The tube was left to dry horizontally on its stem for 48 hours at 4°C and then for 24 hours at 50°C. It was rehydrated with PBS 1X, detached from the stem after about 20 minutes and rinsed 3 times with PBS 1X. The PVA tube was stored in 1X PBS at 4°C protected from light.

### X-ray Powder Diffraction (XRPD)

The equipment used for the measurement is an INEL-THERMO FISHER SCINTIFIC EQUINOX 1000 X-ray diffractometer with asymmetric geometry. The incident monochromatic X-ray beam (Cu Kα1 radiation: 0.154056 nm) makes an angle of about 6° with the sample. Two crossed slits (0.10 × 5 mm²) are placed in the beam 70 mm in front of the sample. The profile is collected on a curved linear detector (0°-113°) placed 180 mm from the sample. In order to characterise the phase of the PVA (lattice parameters, microstructure), a Rietveld refinement was performed using MAUD software (Material Analysis Using Diffraction, University of Trento, Trento, Italy).

### Results

To characterize the PVA tubes, the swelling ratio, thickness, crystallinity and morphology of the intraluminal and extraluminal surface were determined.

### Characterization of the PVA tubular hydrogels

### X-ray Powder Diffraction

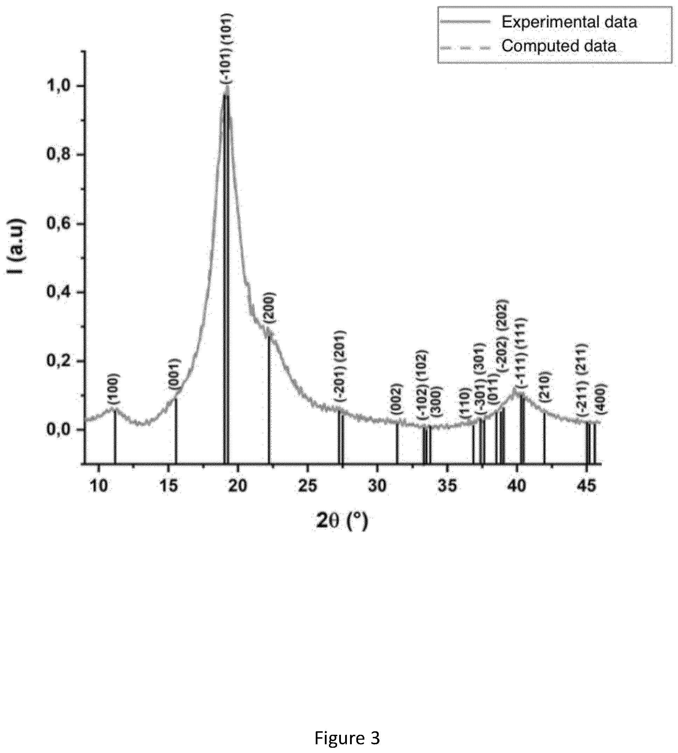

The XRPD profile of a physically reticulated PVA tube is shown in Figure 3. The diffraction pattern of the PVA tube shows several peaks including an intense and broad peak at 19°, indicating the presence of crystalline zones. The resulting pattern is processed by a Rietveld refinement and perfectly matches with a P21/m:b space group, which determines lattice parameters a, b and c of 7.942 ± 0.006 Å, 2.557 ± 0.002 Å and 5.683 ± 0.003 Å respectively. The analysis reveals a semicrystalline structure with nanometric crystallites having a size of 4.45 ± 0.02 nm.

### Example 2: Amination of the intraluminal surface of the PVA tubular hydrogels

In this Example, the experimental protocol for the chemical modification of the intraluminal surface of the PVA tubular hydrogels is described. The chemical modification consists in the amination of the intraluminal surface of the PVA tubes, in order to functionalize the surface with primary amine groups which are required for the subsequent cross-linking of carboxymethylated dextran, low molecular weight heparin or a mixture of both (Example 3). The aminated PVA tubes are characterized by elemental analysis to assess the amount of grafted amines after 1, 2, 3 and 4 successive aminations as well as the uniform distribution of the grafted amines in the aminated PVA tubes.

### Materials and Methods

Materials: 4-aminobutyraldehyde diethyl acetal (4-ABA) was purchased from Acros Organics. The water used, called "ultrapure", was purified at 18,2 MΩ·cm with the Purelab Flex system.

Protocol for the amination of the intraluminal surface of the PVA tubular hydrogels: The luminal surface of the PVA tube was functionalised by closed circulation of a 4-ABA solution using a peristaltic pump. Up to 3 tubes in series were mounted on the system. A 0.83 M aqueous solution of 4-ABA at pH < 1 was prepared.. The 4-ABA solution was introduced into the system (tubing + PVA tube) and circulated at a rate of 10 mL/hr at room temperature for 10 or 30 min in a closed circuit. The PVA tube was then rinsed with PBS 1X with the same peristaltic system principle (open circuit) at a flow rate of 20 mL/h. This protocol was repeated until 2, 3 or 4 successive aminations were achieved.

### Results

### Characterization of the aminated PVA tubes

### Elemental analysis to measure the amount of nitrogen after amination

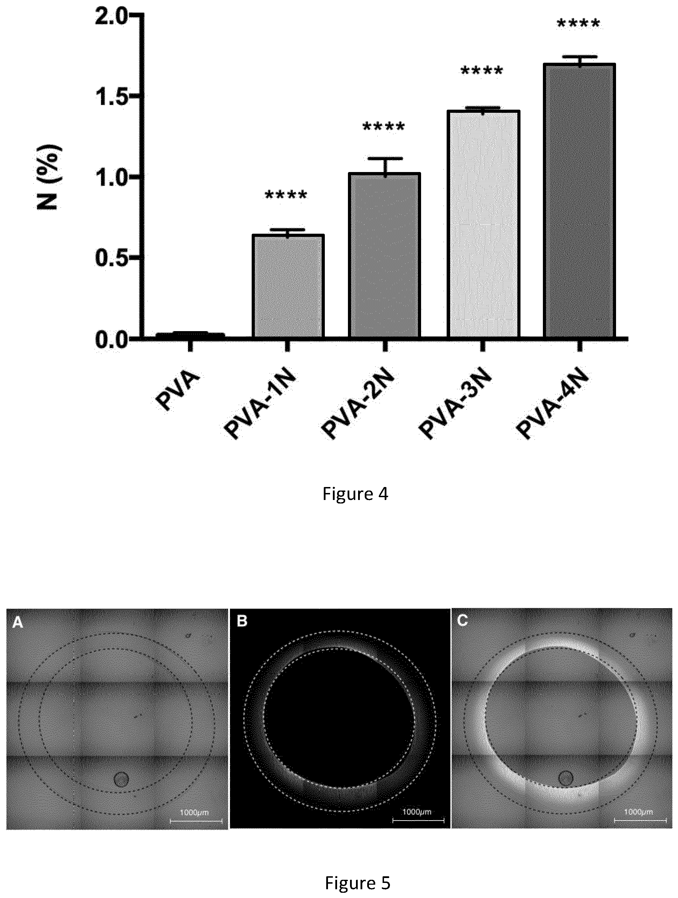

The aminated PVA tubes were characterized by elemental analysis to assess the amount of grafted amines after 1, 2, 3 and 4 successive aminations. The results are shown in Figure 4.

The amount of nitrogen in the unmodified PVA is zero and increases with each amination. After 1, 2, 3 and 4 successive aminations, the PVA tubes show 0.62 ± 0.06, 0.97 ± 0.15, 1.38 ± 0.05 and 1.69 ± 0.09% respectively. The amount of nitrogen increases well with the number of aminations.

### Example 3: Cross-linking of aminated PVA tubular hydrogels with carboxymethylated dextran (CMD), low molecular weight heparin or a mixture of both

In this example, the protocol for the covalent cross-linking of carboxymethylated dextran, low molecular weight heparin or a mixture of both is reported. This example describes the cross-linking of carboxymethylated dextran and heparin to the aminated PVA tubes via the formation of amide bonds between the primary amine groups (introduced on the surface of the PVA, Example 2) and the carboxylic groups present on the dextran and on the heparin. The amine bond formation is done in the presence of a coupling agent, which is required to activate the carboxylic groups present on the dextran and on the heparin, prior to the reaction with the amino groups. In particular, the carboxylic functions of the carboxymethylated dextran and of the low molecular weight heparin are cross-linked using the classical coupling system EDC (1-ethyl-3-(3-dimethylaminopropyl)carbodiimide)/NHS (N-hydroxysuccinimide). The functionalized tubes are characterized by elemental analysis and by analysis of the sulphate content to verify the cross-linking of heparin to the intraluminal surface, as well as by fluorescence microscopy to verify the efficacy of the cross-linking of CMD, by assessing the graft flow resistance to verify the efficacy of the amination and the resistance of the grafted molecules to a determined flux, and by mechanical tests (Example 4).

### Materials and Methods

Materials: The low molecular weight heparin or Tinzaparin (6.5 kDa, 0.5 COOH/glycosidic unit) used was purified from Innohep ^{®} 18000 IU/0.9mL. The 1-ethyl-3-(3-dimethylaminopropyl)carbodiimide (EDC) chlorohydrate was obtained from Carl Roth. The N-hydroxysuccinimide (NHS) was obtained from Thermo Fisher Scientific. The water used, called "ultrapure", was purified at 18.2 MΩ·cm with the Purelab Flex system.

Protocol for the covalent cross-linking of the luminal surface of aminated PVA tubes: Three types of covalent cross-linking were carried out: carboxymethyldextran cross-linking, heparin cross-linking and cross-linking with 50% CMD and 50% (mol COOH/mol COOH) heparin. A solution of the molecule to be cross-linked, CMD or heparin, was prepared with a COOH concentration of 2.82·10⁻² M, corresponding to a concentration of 6.09·10⁻⁴ M in heparin or 1.2·10⁻¹ M in CMD. For 50/50 CMD/heparin cross-linking, the solution was prepared with 3.4·10⁻⁴ M heparin and 6.0·10⁻² M CMD. To assess the cross-linking efficiency, CMD-FITC (CarboxyMethylated Dextran-Fluorescein IsoThioCyanate) was used and the tubes were observed by confocal microscopy. EDC and NHS were added to the CMD or heparin solution at a concentration of 0.13 M and 0.22 M, respectively. The solution was left to stir at room temperature for 30 min before being introduced into the peristaltic pump system in the same way as the 4-ABA solution during amination (Example 2). The solution was circulated at a rate of 10 mL/h overnight at room temperature in closed circuit. The grafted tube was then rinsed with PBS 1X in open circuit at a flow rate of 20 mL/h for at least 2 hours.

### Results

### Characterization of the cross-linked PVA tubular hydrogels

### Fluorescence microscopy measurement to assess the efficacy of the cross-linking of CMD-FITC on the aminated tubular PVA hydrogel

In order to verify the efficiency of the protocol, CMD-FITC was used at 5% (mol/mol) of the total amount of CMD in the reaction solution. The presence or absence of CMD on the luminal surface of the PVA gels was thus validated by confocal microscopy (Figure 5). After rinsing in an open circuit to remove unbound CMD-FITC, all the tubes show fluorescence within the wall except for the non-aminated tube. This confirms that the grafting protocol is effective.

### Example 4: Mechanical and biological characterisation of the PVA, aminated PVA and PVA cross-linked with CMD and heparin tubes

In this Example, the mechanical and biological characterisation of the PVA, aminated PVA and PVA cross-linked with CMD and heparin tubes is reported. Concerning the mechanical properties, the suturability and the compliance of the tubes were considered as the two most important mechanical parameters. The suturability of the tubes quantifies the possibility of effectively joining the prosthesis to sections of healthy vessels by two anastomoses. The compliance evaluates the mechanical response of the vascular tubes in a certain pressure range (in particular, between the systolic and the diastolic pressures). Concerning the biological properties, an estimation of the hemo-compatibility of the prostheses was performed by hemolysis and platelet activation tests as these two mechanisms constitute two forms of toxicity that are prohibitive for the development of vascular prostheses.

### Materials and Methods

### Measurements of the mechanical and biological properties

### Suturability

A suture is made with 6-0 Prolene suture 2-3 mm from the edge of the tube. A container, connected to the suture, is filled with water until the tube ruptures. The mass required for rupture is weighed. The retention force of the suture (*FRS*) can be calculated by the equation : $FRS = \frac{{force}_{sample}}{{d}_{thread} \times {thickness}_{sample}}$ where *FRS* is measured in N/mm², *forceₛₐₘₚₗₑ* is the maximum force the sample can withstand deduced from the supported mass, *d_{thread}* is the diameter of the thread used for the suture and *thicknessₛₐₘₚₗₑ* is the thickness of the sample being tested.

### Compliance

Compliance is measured through a tubing circuit connecting a catheter pump, a manometer and the PVA tube clamped with a three-way valve. Measurements are made in PBS 1X at 37°C using a recirculation pump. The circuit is filled with a PBS 1X solution stained with blue dextran. The staining of the solution allows the detection of leaks or air bubbles in the system that could alter the application of an accurate and constant pressure. The pressure is applied in the circuit by the catheter pump. The diameter of the PVA tube is measured by the high-resolution ultrasound scanner VEVO 2100 (Visualsonics) at the Cochin Institute's Life Imaging Platform. A probe (50MHz) is placed perpendicularly above the sample to obtain a cross-section. The image of the cross-section of the tube is taken when the desired pressure is reached and remains constant. The compliance, measured in %/100mmHg, is calculated by the following equation: $C = \frac{\left({DI}_{P 2}-{DI}_{P 1}\right) / {DI}_{P 1}}{{P}_{2} - {P}_{1}} \times {10}^{4}$ where *DI*_{*P*1} and *DI*_{*P*2} are the internal diameters measured at *P*₁ and*P*₂ pressures respectively, *P*₂ being the highest value.

### Hemolysis

The hemolytic capacity of the tubes is assessed by an hemolysis test with human red blood cells (RBCs). The RBCs are separated from the plasma by centrifuging the whole blood for 12 min at 200 G. A suspension of red cells is prepared by diluting RBCs 1:50 with PBS 1X. PBS 1X and triton X-100 are used as negative and positive controls, respectively. 200 µL of the RBC suspension is injected into the clamped tubes. Samples are incubated for 1 h at 37°C with orbital shaking and then centrifuged for 5 min at 500 G. The absorbance at 540 nm of the supernatants is measured by UV-visible spectrometry. The hemolysis rate (%) is calculated by the equation : $Hemolysis rate \left(%\right) = \frac{{A}_{sample} - {A}_{negative control}}{{A}_{positive control} - {A}_{negative control}} \times 100$

### Results

### Mechanical characterisation

The mechanical properties of PVA, aminated PVA and PVA grafted with CMD and heparin tubes were evaluated.

### Suturability

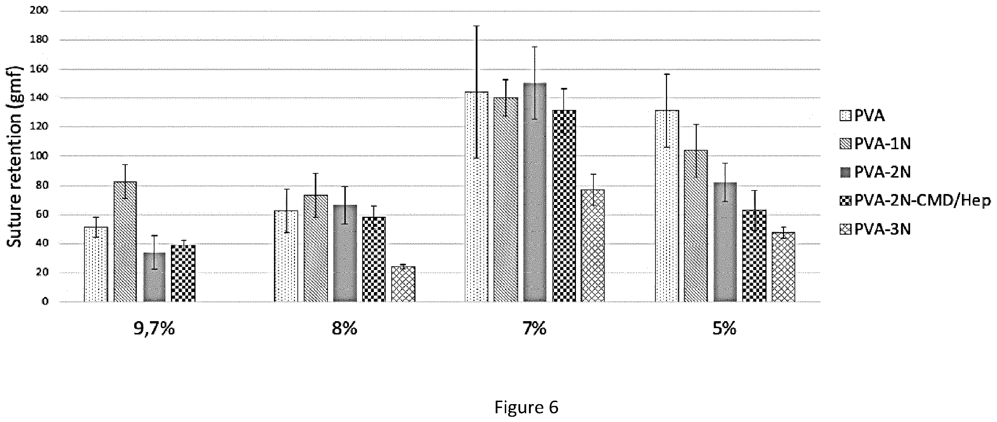

Figure 6 shows the results of the suturability tests carried out on tubes prepared with various PVA concentrations and different chemical modifications of the luminal surface.

According to the literature, the limit of suture retention is fixed at 50gmf because it is considered the minimum value to limit any surgical complications related to anastomosis.

The suture retention of the cross-linked or not cross-linked PVA tubes varies between 24 to 150gmf, depending on their formulation. In particular, the suturability of the tube depends on the PVA concentration, the NaOH concentration and the thickness of the tube wall.

Concerning the effect of the PVA concentration, it is observed that a decrease in the PVA concentration of the tubes leads to an increase of the suturability.

Concerning the effect of the NaOH concentration, it is observed that the suturability is significantly different between tubes formulated with 0.2 and 0.4 M NaOH. This result can be explained by different degrees of acetylation resulting in a difference in cross-linking density and therefore swelling rate. The tube obtained with 0.2 M NaOH is less deacetylated during crosslinking, whereas the tube prepared with 0.4 M NaOH is completely deacetylated. Therefore, the decrease of PVA as well as NaOH concentration has the same effect, which is the increase of the suturability.

Chemical changes in the PVA tubes, on the other hand, lead to a slight decrease in the mass supported by the stitch at the end of the tubes.

Despite the observed slight decrease of suturability in the functionalized tubes, all the samples, except the tubes formulated with 9.7% PVA and 0.2 M NaOH, supported a mass of more than 50gmf and therefore have more than sufficient suturability to be implanted.

### Compliance

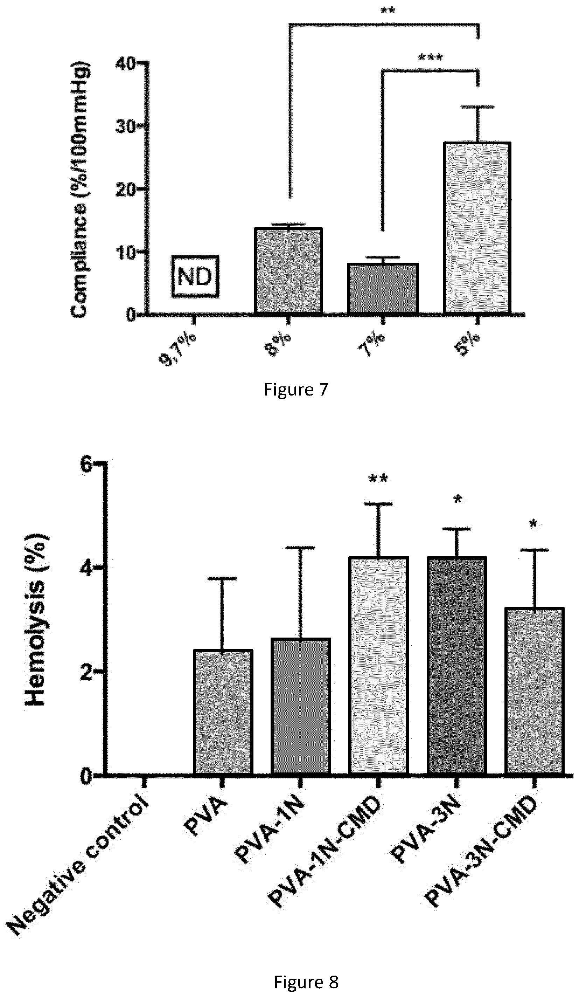

Compliance is assessed using high resolution ultrasound imaging, by placing the sample in a pressure-controlled circuit using a balloon catheter pump and a manometer to vary the pressure from 60 to 140mmHg. The diameter is measured from the ultrasound image of the tube section. The compliance results are shown in Figure 7 as a function of the PVA concentration of the tubes.

Compliance of the not aminated and not cross-linked PVA tubes varies between 2 and 60%/100mmHg depending on the PVA concentration of the tubes and on their thickness. Tubes formulated with 5% (w/w) PVA have the highest compliance of 27.5 ± 5.6%/100mmHg. The compliance of tubes formulated with 8 and 7% PVA is 13.3 ± 1.0%/100mmHg and 7.8 ± 1.3%/100mmHg, respectively. The compliance of tubes formulated with 9.7% (w/w) PVA could not be determined as the burst pressure was reached before 60 mmHg.

The compliance of the tubes formulated with 8% and 7% (w/w) PVA appears to be the most interesting in that these are the tubes whose compliance most closely matches the compliance of small-diameter native arteries, which is between 11 and 15%/100mmHg. For example, tubes formulated with an 8.13%(w/v) PVA solution, molded with 1.2mm internal diameter and having 0.9mm thickness, show a compliance of 14.8%/100mmHg. The compliance of tubes formulated with 7% PVA is slightly lower with respect to the compliance of the coronary arteries and the internal mammary artery, but still well above the compliances of PET and ePTFE prosthesis.

### Flow graft resistance

CMD-graft resistance to physiological flow was tested, namely a 70 mL/min PBS flow was maintained inside FITC-CMD grafted tubes during 35h at 37°C using a peristaltic pump. Two samples were observed under confocal microscopy: one aminated three times, then covalently linked to CMD, another aminated four times, then non-covalently linked to CMD (without EDC). Both samples were found to possess similar fluorescence before and after 35h PBS flow, showing a good flow resistance, whatever the graft technique.

### Biological characterization

The biological properties of PVA tubes and of PVA tubes cross-linked with CMD were evaluated. In what follows, the results obtained by measuring the hemolysis induced by the PVA tubes are reported.

### Hemolysis

The hemolytic effects of the luminal surface of the PVA tubes and that of the PVA tubes cross-linked with CMD were evaluated. The results are shown in Figure 8.

The results are expressed as a percentage of hemolysis compared to the positive control. Hemolysis induced varies between 2.3 to 4.1%. The PVA tube and the one time aminated PVA tube (PVA-1) are considered slightly hemolytic as they induce between 2 and 5% hemolysis (according to Assessment of Hemolytic Properties of Materials from the American Society for Testing and Materials ASTM F756-00, 2000 and to ISO 10993-4). The PVA-1N-CMD, PVA-3N and PVA-3N-CMD tubes are considered hemolytic as they induce more than 5% haemolysis. As the hemolysis induced by the samples is less than or equal to about 5%, the tubes remain within the accepted standards.

### Abbreviations used in this text:

4-ABA : 4-AminoButyraldehyde diethyl Acetal
CMD: CarboxyMethylated Dextran
CMD-FITC : CarboxyMethylated Dextran-Fluorescein IsoThioCyanate
EDC: 1-Ethyl-3-(3-Dimethylaminopropyl)Carbodiimide
ePTFE : Expanded PolyTetraFluoroEthylene
FRS : Retention Force of the Suture
MW : Molecular Weight
NHS: N-HydroxySuccinimide
PBS: Phosphate-Buffered Saline
PET : PolyEthylene Terephthalate
PVA: PolyVinyl Alcohol
RBC : Red Blood Cell
SEM : Scanning Electron Microscopy
UP: UltraPure water
XRPD : X-Ray Powder Diffraction

## Claims

1. A method of obtaining an artificial vascular prosthesis, comprising the steps of:
a. providing:
i. a polyvinyl alcohol (PVA) aqueous solution wherein the PVA has a deacetylation level between 80 and 90%, preferably between 85 and 88% and a molecular mass in the range between 85 and 130 kDa ;
ii. an alcaline aqueous solution ;
b. mixing the PVA and the alcaline solutions so that the final concentration of PVA is in the range from 4 to 10% w/w, preferably between 7.5 and 8.5% w/w, and the final concentration of the OH⁻ is in the range from 0.15 M to 0.30 M, so as to obtain a homogenous bubble-free viscous solution ;
c. introducing the solution obtained in step b into a mold comprising two coaxial cylinders, wherein the external diameter (d1) of the smaller cylinder is in the range from 0.8 to 6.0 mm, the internal diameter (d2) of the larger cylinder is in the range from 3.0 to 9.0 mm and the thickness (d2-d1) is in the range from 0.9 to 3.0 mm, preferably between 1.5 and 2.5 mm;
d. freezing the solution in the mold to obtain a tube of frozen PVA hydrogel;
e. removing the part of the mold comprising the larger cylinder;
f. drying the tube for at least 24 hours, preferably 24-72 hours at a temperature in the range from 4°C to 20°C, preferably in the range from 4°C to 10°C and for 18-36 hours at a temperature in the range from 30 to 60°C, preferably around 50°C ;
g. placing the tube in a buffer at room temperature for at least 10 minutes ; and
h. removing the part of the mold comprising the smaller cylinder.

2. The method of claim 1, wherein the tube obtained in step h has a compliance in the range from 7 to 18%, preferable from 11 to 15% as assessed by measuring the internal diameter while pressures ranging from 60 mmHg to 140 mmHg are applied.

3. The method of claim 1 or claim 2, further comprising a step of contacting the intraluminal surface of the tube with the solution of an amination reagent to covalently link primary amine groups to said intraluminal surface.

4. The method of claim 3, wherein the amination of the intraluminal surface of the tube is performed by contacting the intraluminal surface of the tube with a solution of 4-aminobutyraldehyde diethyl acetal (4-ABA), preferably at a concentration in the range of 0.5 to 2.0 M, preferably 0.8 to 1 M, at pH < 1.

5. The method of claim 3 or claim 4, wherein the amination of the intraluminal surface of the tube is performed by circulating, a solution of the amination reagent, followed by the rinsing of the intraluminal surface of the tube.

6. The method of claim 5, wherein several cycles of circulating the solution of the amination reagent, followed by the rinsing of the intraluminal surface of the tube, are performed.

7. The method of claims 3 to 6, further comprising a step of functionalizing the intraluminal surface of the tube by linking molecules having a therapeutic interest to said surface.

8. The method of claim 7, wherein said molecules having a therapeutic interest are selected from the group consisting of: anti-thrombogenic agents, anti-bacterial agents, anti-viral agents, growth factors, cytokines and mixtures thereof.

9. The method of claims 7 or 8, wherein said molecules having a therapeutic interest comprise carboxymethyldextran and/or heparin.

10. The method of any of claims 7 to 9, wherein the functionalization is performed by a coupling system.

11. The method of any of claims 9 to 10, wherein carboxymethyldextran and heparin are covalently linked to the aminated intraluminal surface in a ratio between 1:2 and 2:1, preferably in a ratio1 : 1.

12. The method of any of claims 7 to 11, wherein the functionalization of the aminated intraluminal surface of the tube is performed by circulating a solution of the molecule of therapeutic interest, followed by the rinsing of the intraluminal surface of the tube.

13. A PVA tube or a functionalized vascular prosthesis obtained by the method according to any of claims 1 to 12.

14. The PVA tube or functionalized vascular prosthesis according to claim 13, having a compliance in the range between 11 and 15%/100mmHg as assessed by measuring the internal diameter while pressures ranging from 60 mmHg to 140 mmHg are applied.

15. The PVA tube or functionalized vascular prosthesis of claims 13 or 14, **characterized by** an induced hemolysis below 5%, preferably below 2%.

## Patentansprüche

1. Verfahren zum Erhalten einer künstlichen Gefäßprothese, umfassend die folgenden Schritte:
a. Bereitstellen:
i. einer wässrigen Polyvinylalkohol (PVA)-Lösung, wobei der PVA einen Desacetylierungsgrad zwischen 80 und 90 %, vorzugsweise zwischen 85 und 88 % und eine Molekülmasse im Bereich zwischen 85 und 130 kDa aufweist;
ii. einer wässrigen alkalischen Lösung;
b. Mischen des PVA und der alkalischen Lösungen, so dass die Endkonzentration von PVA im Bereich von 4 bis 10 % G/G, vorzugsweise zwischen 7,5 und 8,5 % G/G, liegt und die Endkonzentration von OH⁻ im Bereich von 0,15 M bis 0,30 M liegt, um eine homogene, blasenfreie, viskose Lösung zu erhalten;
c. Einführen der in Schritt b erhaltenen Lösung in eine Form, die zwei koaxiale Zylinder umfasst, wobei der Außendurchmesser (d1) des kleineren Zylinders im Bereich von 0,8 bis 6,0 mm liegt, der Innendurchmesser (d2) des größeren Zylinders im Bereich von 3,0 bis 9,0 mm liegt und die Dicke (d2-d1) im Bereich von 0,9 bis 3,0 mm, vorzugsweise zwischen 1,5 und 2,5 mm, liegt;
d. Gefrieren der Lösung in der Form, um einen Schlauch aus gefrorenem PVA-Hydrogel zu erhalten;
e. Entfernen des Teils der Form, der den größeren Zylinder umfasst;
f. Trocknen des Schlauchs für mindestens 24 Stunden, vorzugsweise 24-72 Stunden bei einer Temperatur im Bereich von 4 °C bis 20 °C, vorzugsweise im Bereich von 4 °C bis 10 °C und für 18-36 Stunden bei einer Temperatur im Bereich von 30 bis 60 °C, vorzugsweise um 50 °C;
g. Einlegen des Schlauchs in einen Puffer bei Raumtemperatur für mindestens 10 Minuten; und
h. Entfernen des Teils der Form, der den kleineren Zylinder umfasst.

2. Verfahren nach Anspruch 1, wobei der in Schritt h erhaltene Schlauch eine Compliance im Bereich von 7 bis 18 %, vorzugsweise von 11 bis 15 % aufweist, wie durch Messen des Innendurchmessers bewertet, während Drücke im Bereich von 60 mmHg bis 140 mmHg angelegt werden.

3. Verfahren nach Anspruch 1 oder Anspruch 2, ferner umfassend einen Schritt des Inkontaktbringens der intraluminalen Oberfläche des Schlauchs mit der Lösung eines Aminierungsreagenzes, um primäre Amingruppen kovalent an die genannte intraluminale Oberfläche zu binden.

4. Verfahren nach Anspruch 3, wobei die Aminierung der intraluminalen Oberfläche des Schlauchs durch Inkontaktbringen der intraluminalen Oberfläche des Schlauchs mit einer Lösung von 4-Aminobutyraldehyddiethylacetal (4-ABA) durchgeführt wird, vorzugsweise bei einer Konzentration im Bereich von 0,5 bis 2,0 M, vorzugsweise 0,8 bis 1 M, bei einem pH < 1.

5. Verfahren nach Anspruch 3 oder Anspruch 4, wobei die Aminierung der intraluminalen Oberfläche des Schlauchs durch Zirkulierenlassen einer Lösung des Aminierungsreagenzes, gefolgt von dem Spülen der intraluminalen Oberfläche des Schlauchs, durchgeführt wird.

6. Verfahren nach Anspruch 5, wobei mehrere Zyklen des Zirkulierenlassens der Lösung des Aminierungsreagenzes, gefolgt von dem Spülen der intraluminalen Oberfläche des Schlauchs, durchgeführt werden.

7. Verfahren nach den Ansprüchen 3 bis 6, ferner umfassend einen Schritt des Funktionalisierens der intraluminalen Oberfläche des Schlauchs durch Binden von Molekülen mit einem therapeutischen Interesse an die genannte Oberfläche.

8. Verfahren nach Anspruch 7, wobei die genannten Moleküle mit einem therapeutischen Interesse ausgewählt sind aus der Gruppe bestehend aus: antithrombogenen Mitteln, antibakteriellen Mitteln, antiviralen Mitteln, Wachstumsfaktoren, Zytokinen und Mischungen davon.

9. Verfahren nach den Ansprüchen 7 oder 8, wobei die genannten Moleküle mit einem therapeutischen Interesse Carboxymethyldextran und/oder Heparin umfassen.

10. Verfahren nach einem der Ansprüche 7 bis 9, wobei die Funktionalisierung durch ein Kopplungssystem durchgeführt wird.

11. Verfahren nach einem der Ansprüche 9 bis 10, wobei Carboxymethyldextran und Heparin kovalent an die aminierte intraluminale Oberfläche in einem Verhältnis zwischen 1:2 und 2:1, vorzugsweise in einem Verhältnis von 1:1, gebunden werden.

12. Verfahren nach einem der Ansprüche 7 bis 11, wobei die Funktionalisierung der aminierten intraluminalen Oberfläche des Schlauchs durch Zirkulierenlassen einer Lösung des Moleküls von therapeutischem Interesse, gefolgt von dem Spülen der intraluminalen Oberfläche des Schlauchs, durchgeführt wird.

13. PVA-Schlauch oder eine funktionalisierte Gefäßprothese, erhalten durch das Verfahren nach einem der Ansprüche 1 bis 12.

14. PVA-Schlauch oder funktionalisierte Gefäßprothese nach Anspruch 13, mit einer Compliance im Bereich zwischen 11 und 15 %/100 mmHg, wie durch Messen des Innendurchmessers bewertet, während Drücke im Bereich von 60 mmHg bis 140 mmHg angelegt werden.

15. PVA-Schlauch oder funktionalisierte Gefäßprothese nach den Ansprüchen 13 oder 14, **gekennzeichnet durch** eine induzierte Hämolyse von unter 5 %, vorzugsweise unter 2 %.

## Revendications

1. Procédé d'obtention d'une prothèse vasculaire artificielle, comprenant les étapes consistant à :
a. fournir :
i. une solution aqueuse d'alcool polyvinylique (PVA) dans laquelle le PVA a un taux de désacétylation compris entre 80 et 90 %, de préférence entre 85 et 88 % et une masse moléculaire dans la plage comprise entre 85 et 130 kDa ;
ii. une solution aqueuse alcaline ;
b. mélanger le PVA et les solutions alcalines de sorte que la concentration finale de PVA soit dans la plage de 4 à 10 % p/p, de préférence entre 7,5 et 8,5 % p/p, et que la concentration finale de OH⁻ soit dans la plage de 0,15 M à 0,30 M, de manière à obtenir une solution visqueuse homogène et exempte de bulles ;
c. introduire la solution obtenue à l'étape b dans un moule comprenant deux cylindres coaxiaux, dans lequel le diamètre externe (d1) du plus petit cylindre est dans la plage de 0,8 à 6,0 mm, le diamètre interne (d2) du plus grand cylindre est dans la plage de 3,0 à 9,0 mm et l'épaisseur (d2-d1) est dans la plage de 0,9 à 3,0 mm, de préférence entre 1,5 et 2,5 mm ;
d. congeler la solution dans le moule pour obtenir un tube d'hydrogel de PVA congelé ;
e. retirer la partie du moule comprenant le plus grand cylindre ;
f. sécher le tube pendant au moins 24 heures, de préférence 24-72 heures à une température dans la plage de 4 °C à 20 °C, de préférence dans la plage de 4 °C à 10 °C et pendant 18-36 heures à une température dans la plage de 30 à 60 °C, de préférence autour de 50 °C ;
g. placer le tube dans un tampon à température ambiante pendant au moins 10 minutes ; et
h. retirer la partie du moule comprenant le plus petit cylindre.

2. Procédé selon la revendication 1, dans lequel le tube obtenu à l'étape h a une compliance dans la plage de 7 à 18 %, de préférence de 11 à 15 % telle qu'évaluée par la mesure du diamètre interne tandis que des pressions allant de 60 mmHg à 140 mmHg sont appliquées.

3. Procédé selon la revendication 1 ou la revendication 2, comprenant en outre une étape de mise en contact de la surface intraluminale du tube avec la solution d'un réactif d'amination pour lier de manière covalente des groupes amine primaire à ladite surface intraluminale.

4. Procédé selon la revendication 3, dans lequel l'amination de la surface intraluminale du tube est réalisée en mettant en contact la surface intraluminale du tube avec une solution de 4-aminobutyraldéhyde diéthyl acétal (4-ABA), de préférence à une concentration dans la plage de 0,5 à 2,0 M, de préférence de 0,8 à 1 M, à un pH < 1.

5. Procédé selon la revendication 3 ou la revendication 4, dans lequel l'amination de la surface intraluminale du tube est réalisée en faisant circuler une solution du réactif d'amination, suivie par le rinçage de la surface intraluminale du tube.

6. Procédé selon la revendication 5, dans lequel plusieurs cycles de circulation de la solution du réactif d'amination, suivis par le rinçage de la surface intraluminale du tube, sont réalisés.

7. Procédé selon les revendications 3 à 6, comprenant en outre une étape de fonctionnalisation de la surface intraluminale du tube en liant des molécules ayant un intérêt thérapeutique à ladite surface.

8. Procédé selon la revendication 7, dans lequel lesdites molécules ayant un intérêt thérapeutique sont choisies dans le groupe constitué par : les agents antithrombogènes, les agents antibactériens, les agents antiviraux, les facteurs de croissance, les cytokines et leurs mélanges.

9. Procédé selon la revendication 7 ou 8, dans lequel lesdites molécules ayant un intérêt thérapeutique comprennent du carboxyméthyldextrane et/ou de l'héparine.

10. Procédé selon l'une quelconque des revendications 7 à 9, dans lequel la fonctionnalisation est réalisée par un système de couplage.

11. Procédé selon l'une quelconque des revendications 9 à 10, dans lequel le carboxyméthyldextrane et l'héparine sont liés de manière covalente à la surface intraluminale aminée dans un rapport compris entre 1:2 et 2:1, de préférence dans un rapport de 1:1.

12. Procédé selon l'une quelconque des revendications 7 à 11, dans lequel la fonctionnalisation de la surface intraluminale aminée du tube est réalisée en faisant circuler une solution de la molécule d'intérêt thérapeutique, suivie par le rinçage de la surface intraluminale du tube.

13. Tube en PVA ou prothèse vasculaire fonctionnalisée obtenu(e) par le procédé selon l'une quelconque des revendications 1 à 12.

14. Tube en PVA ou prothèse vasculaire fonctionnalisée selon la revendication 13, ayant une compliance dans la plage comprise entre 11 et 15 %/100 mmHg telle qu'évaluée par la mesure du diamètre interne tandis que des pressions allant de 60 mmHg à 140 mmHg sont appliquées.

15. Tube en PVA ou prothèse vasculaire fonctionnalisée des revendications 13 ou 14, caractérisé(e) par une hémolyse induite inférieure à 5 %, de préférence inférieure à 2 %.
